# EUROPEAN PATENT APPLICATION

(11) **EP 4 736 858 A1**
(43) Date of publication of application: **06.05.2026**
(21) Application number: 24210453.7
(22) Date of filing: 04.11.2024
(51) Int. Cl.: A61K 31/66, A61P 3/00, A61P 3/04

(54) **TETRAORGANOPHOSPHONIUM COMPOUNDS FOR USE IN THE PREVENTION AND TREATMENT OF METABOLIC DISEASES**

(71) Applicant: Institiute of Clinical and Experimental Medicine, 14021 Praha 4 (CZ)
(72) Inventor: Stemberkova-Hubackova, Sona, 14900 Praha 4 (CZ); Stursa, Jan, 14800 Praha 4 (CZ); Werner, Lukas, 25169 Velke Popovice - Brtnice (CZ); Haluzik, Martin, 12000 Praha 2 (CZ)
(74) Representative: Harber IP s.r.o.

(57) **Abstract**

The present invention relates to the use of compounds of formula I 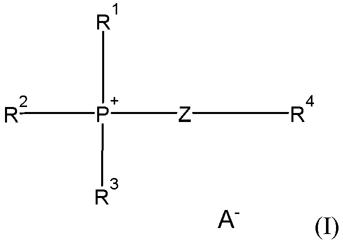 for treatment of metabolic disease,
wherein R¹, R², R³ are independently selected from the group consisting of phenyl, benzyl, and cyclohexyl; Z is a linear alkylene, alkenylene or alkynylene chain with the number of carbon atoms in the range of from 6 to 20, wherein one or more -CH₂- groups may optionally be substituted with one or more O atoms; R⁴ is selected from the group consisting of H, OH, halogen atom, and (C1 to C10)alkoxy group, which may optionally be substituted with one or more -OH groups; and A⁻ is a pharmaceutically acceptable anion of an organic or inorganic acid.

## Description

### Field of Art

The present invention is related to the use of phosphonium compounds for the treatment of metabolic diseases, in particular obesity and type 2 diabetes mellitus and their complications like liver steatosis, chronic/diabetic kidney disease, cardiovascular diseases, pancreatic tumour, pancreatic fibrosis, and/or pulmonary fibrosis.

### Background Art

The increasing prevalence of metabolic diseases and their chronic debilitating complications represent one of the most significant health threats worldwide. Obesity, type 2 diabetes mellitus (T2DM), and its complications, currently affecting 422 million people worldwide, are expected to become the seventh leading cause of death by 2030. This is particularly evident in elderly patients, where it can affect as many as 30-40 % of the population, compared to approximately 6-25 % of patients under 65 years of age.

Bariatric surgery (also called metabolic surgery, reflecting its outstanding beneficial metabolic effects) is currently the most efficient approach for the treatment of obesity, resulting in substantial and sustained weight loss and improvement of metabolic complications of obesity. In addition to its weight-reducing effects, bariatric surgery markedly improves diabetes control, with a high percentage of patients achieving diabetes remission. The improvement of metabolic health goes beyond the expected effect of weight loss suggesting the involvement of other mechanisms in addition to weight reduction triggered by bariatric surgery including alleviation of low-grade inflammation, alterations in gut hormone secretion profiles and bile acids, neural mechanisms as well as changes in gut microbiota. However, none of these mechanisms can fully explain the profound and long-lasting metabolic effects of bariatric surgery.

Cellular senescence can occur in all dividing cells in response to excessive extracellular or intracellular stress, leading to cell cycle arrest. Despite being locked in cell cycle arrest, senescent cells remain metabolically active and secrete a wide range of pro-inflammatory cyto- and chemokines, growth factors and extracellular matrix modulators collectively called "senescence-associated secretory phenotype" (SASP). SASP is primarily responsible for the attraction and activation of immune cells that remove senescent cells and restore tissue homeostasis. However, the inability of immune cells to eliminate senescent cells may lead to inflammation or the development of age-related diseases. Due to their significant accumulation in the adipose tissue of patients with obesity and T2DM, cellular senescence, with its emerging role in the pathogenesis of obesity, T2DM, and related complications, might offer a novel mechanism underlying bariatric surgery-associated metabolic improvements.

However, not all patients can undergo bariatric surgery, thus relying on pharmacotherapy for obesity, T2DM, and related complications. GLP-1 receptor agonists and SGLT2-inhibitors (gliflozins) currently represent the most promising classes of glucose-lowering therapies, affecting not only glucose control but also chronic complications, at least in part, independently of the improvement of diabetes control. Despite these significant advances, many patients develop chronic complications. Moreover, the landscape of treatment still primarily focuses on symptom management rather than addressing the underlying causes. Novel complementary treatment approaches, such as targeting senescence that can prevent metabolic diseases and their chronic complications, are still needed.

Senolytics reduce the number of senescent cells in an organism. Their positive metabolic effect was suggested based on animal experiments, where the administration of senolytics was associated with improved glucose control and other obesity- and diabetes-related metabolic disturbances (Kulkarni, A.S., et al., Aging Cell, 2018. 17(2), Hickson, L.J., et al., Ebiomedicine, 2019. 47: p. 446-456, Palmer, A.K., et al., Aging Cell, 2019. 18(3)). Clinical testing of the anti-diabetic agent Metformin on human aging and the development of aging-related complications is currently underway (registration number: NCT04264897). Despite these promising results, all currently available agents target specific proteins or pathways that are not expressed in all types of senescent cells. This limited efficacy has prompted a search for novel therapeutic targets.

Mitochondrial dysfunction is an important determinant of the aging process and plays a critical role in the pathogenesis of a wide array of metabolic disorders, impacting energy metabolism, insulin resistance, and accumulation of metabolic intermediates. This has spurred interest in directly targeting mitochondrial dysfunction as a novel therapeutic approach.

Alkyl triphenylphosphonium (ATPP) salts have been utilized in a range of scientific and industrial domains owing to their unique chemical properties. In the realm of biochemistry and cell biology, these compounds are noted for their ability to selectively penetrate mitochondrial membranes, which is attributed to the inherent negative membrane potential of the mitochondria (Liberman, E.A. et al. Nature 1969, 222, 1076-8.). ATPP chains are well-known mitochondria-targeting vectors linked to pharmaceutically active compounds (summarized in Zielonka, Joseph et al. Chem. Rev.2017, 117, 10043-10120). Their application extends to pharmaceuticals, serving as phase transfer catalysts that enhance reaction efficiencies in organic synthesis, and in materials science, where they contribute to the development of novel polymers and coatings with distinctive electrical and optical characteristics.

### Disclosure of the Invention

The present invention relates to analogues of alkyl triphenylphosphonium salts as potential novel medicaments for the treatment of metabolic diseases.

The use of ATPP salts to treat metabolic diseases enhances their capacity for mitochondrial modulation. By fine-tuning the carbon chain length, these compounds effectively target and rectify mitochondrial dysfunction inherent in metabolic disorders. This application represents a significant shift from both the traditional uses of said compounds and existing metabolic disease treatments, where direct mitochondrial targeting remains largely unexplored.

Introducing ATPP salts as a treatment for metabolic diseases builds upon their established scientific and industrial applications, targeting mitochondrial dysfunction at the core of these conditions. This novel therapeutic strategy promises significant advancement in the management of metabolic disorders.

The aim of the present invention is to provide new medicaments for treatment of metabolic diseases and uses for said purpose phosphonium compounds, containing analogues of alkyl triphenylphosphonium cations of general formula I 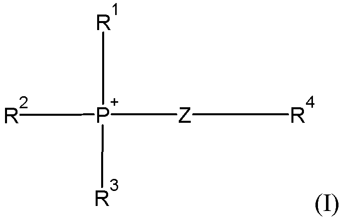 wherein
R¹, R², R³ are independently selected from the group consisting of phenyl, benzyl and cyclohexyl; Z is a linear alkylene, alkenylene or alkynylene chain with the number of carbon atoms in the range of from 6 to 20, wherein one or more -CH₂- groups may optionally be substituted with one or more O atoms;
R⁴ is selected from the group consisting of H, OH, halogen atom, and (C1 to C10)alkoxy group, which may optionally be substituted with one or more -OH groups.

The phosphonium compounds include a counterion A⁻, which may be a pharmaceutically acceptable anion of an organic or inorganic acid, to form a pharmaceutically acceptable salt. The invention also encompasses the pharmaceutically acceptable salts, addition salts and solvates of the above defined phosphonium compounds.

The term "alkyl" means a saturated hydrocarbon chain, which may be straight, branched or cyclic or cycle-containing, and which is derived from an alkane by removal of one hydrogen atom.

The term "alkenyl" means a hydrocarbon chain containing at least one double bond between carbon atoms, and which is derived from an alkene by removal of one hydrogen atom. The hydrocarbon chain may be straight, branched or cyclic or cycle-containing.

The term "alkynyl" means a hydrocarbon chain containing at least one triple bond between carbon atoms, and optionally also one or more double bonds between carbon atoms, and which is derived from an alkyne by removal of one hydrogen atom. The hydrocarbon chain may be straight, branched or cyclic or cycle-containing.

The term "alkylene" means a bivalent saturated linear hydrocarbon chain. The chain has two valencies, i.e. it is derived from an alkane by removal of two hydrogen atoms from different carbon atoms, and it binds as a linker or bridge via two single bonds.

The term "alkenylene" means a bivalent linear hydrocarbon chain containing at least one double bond between carbon atoms, and which is derived from an alkene by removal of two hydrogen atoms from different carbon atoms, and it binds as a linker or bridge via two single bonds.

The term "alkynylene" means a bivalent linear hydrocarbon chain containing at least one triple bond between carbon atoms, and optionally also one or more double bonds between carbon atoms, and which is derived from an alkyne by removal of two hydrogen atoms from different carbon atoms, and it binds as a linker or bridge via two single bonds.

The term "halogen" means F, Cl, Br or I.

The term "alkoxy group" means an alkyl, alkenyl or alkynyl group which is singularly bonded to oxygen. Examples of an alkoxy group may be -O-CH₃, -O-CH₂-CH₃ or -O-CH₂-CH=C(CH₃)₂. The term "obesity" means is a medical condition, in which excess body fat has accumulated to such an extent that it can potentially have negative effects on health. People are classified as obese when their body mass index (BMI) is over 30 kg/m².

The term "overweight" is a medical condition, in which body mass index (BMI) is in the range of from 25 to 30 kg/m².

The term "pharmaceutically acceptable anion" refers to anions that retain the biological effectiveness and properties of the claimed compounds of general formula I according to this invention, and which are within reasonable medical judgment suitable for use in contact with the tissues of humans and lower animals without undue toxicity, irritation, allergic reactions, and the like, and have an acceptable benefit/risk ratio.

The object of the present invention is the use of the above-defined phosphonium compounds as medicaments in the treatment of metabolic diseases, particularly type 2 diabetes mellitus, obesity, and their complications such as chronic/diabetic kidney disease, metabolic dysfunction-associated fatty liver disease, metabolic dysfunction-associated steatohepatitis, cardiovascular diseases (atherosclerosis, cardiac ischemia, heart failure with preserved ejection fraction, heart failure with reduced ejection fraction), chronic respiratory diseases (idiopatic pulmonary disease, chronic obstructive pulmonary disease), neurodegenerative diseases (diabetic neuropathy), and cancer (especially pancreatic cancer).

In some embodiments, R¹, R² and R³ are independently selected from phenyl, and cyclohexyl.

In some embodiments, all of R¹, R² and R³ are the same.

In some embodiments, Z is a linear alkylene linker of formula -(CH₂)ₙ-, or a linear alkenylene linker of formula -(CH₂)ₚ-CH=CH-(CH₂)_{q}-, wherein n is an integer in the range of from 6 to 20, preferably in the range of from 8 to 18, p and q are independently integers in the range of from 1 to 17 with the proviso that the sum of p and q is from 4 to 18, preferably p and q are from 2 to 9, more preferably from 4 to 8; wherein one or more -CH₂- groups may optionally be substituted with one or more O atoms. Preferably, Z is a linear alkylene linker of formula -(CH₂)ₙ-, wherein n is an integer in the range of from 10 to 16, or Z is a linear alkenylene linker of formula -(CH₂)ₚ-CH=CH-(CH₂)_{q}-, wherein p and q are independently integers in the range of from 6 to 8.

In some embodiments, Z is a linear (C6 to C20)alkenylene linker, preferably (C10 to C18)alkenylene linker, wherein one or more -CH₂- groups may optionally be substituted with one or more O atoms. Preferably, said (C6 to C20)alkenylene linker contains one double bond.

In some embodiments, R⁴ is selected from the group consisting of H, OH, and halogen atom.

In some embodiments, R⁴ is a (C6 to C10)alkoxy group, which may optionally be substituted with at least one -OH group.

In one embodiment, the anion present in the salts of the phosphonium compounds of formula I is typically selected from Cl⁻, Br, I⁻, carbonate, bicarbonate, nitrate, sulphate, phosphate, hexafluorophosphate, tetrafluoroborate, acetate, ascorbate, aspartate, benzenesulfonate, benzoate, besylate, bitartrate, camsylate, citrate, decanoate, edetate, esylate, formiate, fumarate, gluceptate, gluconate, glutamate, glycolate, hexanoate, hydroxynaphthoate, isethionate, lactate, lactobionate, malate, maleate, mandelate, mesylate, methyl sulfate, methansulfonate, mucate, napsylate, octanoate, oleate, oxalate, pamoate, pantothenate, polygalacturonate, propionate, salicylate, stearate, succinate, tartrate, teoclate, tosylate. The corresponding acids may form the acid addition salts of the phosphonium compounds of formula I, the use thereof in the treatment of metabolic diseases being also the object of the present invention.

In some embodiments, the anion present in the salts of the phosphonium compounds of formula I is selected from Cl⁻, Br⁻, I⁻, and methansulfonate.

In one specific embodiment, the phosphonium compound of general formula I has R¹, R², and R³ independently selected from phenyl and cyclohexyl, preferably all of R¹, R², and R³ are the same; R⁴ is selected from the group consisting of H, OH, halogen atom; preferably from OH, Cl, and Br; and Z is a linear alkylene or alkenylene linker with the number of carbon atoms in the range of from 10 to 20.

In one specific embodiment of the phosphonium compound of general formula I, Z is selected from -(CH₂)₈-CH=CH-(CH₂)₈-; -(CH₂)ₙ-, wherein n is an integer in the range of from 10 to 16; -C(CH₃)=CH-(CH₂)₈-; -C(CH₃)=CH-CH₂-O-(CH₂)₁₀-; and -(CH₂)₉-O-(CH₂)₈-.

Specifically, the following phosphonium compounds are preferred:
(18-hydroxyoctadec-9-en-1-yl)triphenylphosphonium 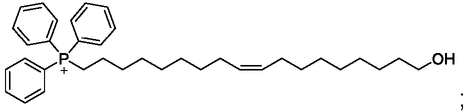
(10-bromodecyl)tricyclohexylphosphonium 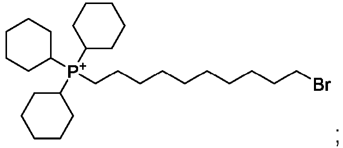
Tricyclohexyl(16-hydroxyhexadecyl)phosphonium methansulfonate 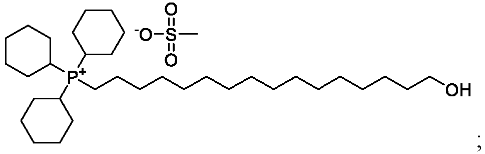 and
(16-hydroxyhexadecyl)triphenylphosphonium

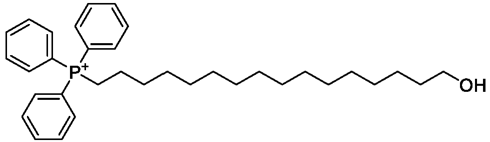

The object of the present invention is also the use of the above-defined phosphonium compounds in a prophylactic treatment, preferably for the prevention of metabolic diseases in individuals suffering from obesity or in overweight individuals.

In one embodiment, the use of the above-defined phosphonium compounds is in a prevention of obesity in overweight individuals.

Another object of the present invention is the use of phosphonium compounds of general formula I as a food supplement to reduce overweight and/or to improve overweight-induced metabolic complications in overweight individuals.

The object of the present invention is also a pharmaceutical composition containing at least one phosphonium compound of general formula I as the active pharmaceutical ingredient (API) in a therapeutically effective amount, and at least one pharmaceutically acceptable excipient, such as a carrier, solvent, filler, binder, disintegrant, coating, glidant, lubricant, preservative, flavor, and colorant. The pharmaceutical composition may be in a liquid or in a solid form. Liquid forms include solutions, suspensions, dispersions, adapted *e.g.* for injection or oral administration. Solid forms include, for example, capsules, tablets, coated tablets, powders, suppositories, and other forms.

The term "therapeutically effective amount" of a compound of the present invention refers to an amount of the compound or drug that is effective in treating a disease or disorder in a human or mammal.

Phosphonium compounds of general formula I according to the present invention are commercially available or they may be prepared by alkylation of halogenide intermediate with corresponding phosphine (triphenylphosphine, tricyclohexyl phosphine and others) under higher temperature (from 60 to 100 °C) in an aprotic solvent, most preferably DMF.

The phosphonium compounds of general formula I or the pharmaceutical composition according to the present invention are also suitable for use in a prophylactic treatment, preferably for the prevention of metabolic diseases in obese patients.

The phosphonium compounds of general formula I or the pharmaceutical composition according to the present invention are also suitable for use in a prevention of metabolic diseases in overweight people.

### Brief description of drawings

Figure 1: Comparison of level of triacylglycerol (TAG) in liver of tested animals accompanied with decreased "ballooning" caused by lipid accumulation.
Figure 2: Graf of KPC-1 tumor growth as comparison of untreated animals and animals treated with Lab210.
Figure 3: Survival graph of animals with KCP- tumor showing prolonged survival of animals treated with Lab210 in comparison with untreated animals.

### Examples

Known or commercially available compounds prepared for the experiments.

### Example 1: (10-((3-methylbut-2-en-1-yl)oxy)decyl)triphenylphosphonium bromide (Lab 167)

Isoprenol was dissolved in DMF (100 mg / 1 ml) and sodium hydride (0.8 equivalent) was added. Reaction mixture was stirred for 1.5 hours and 1,10-dibromodecane (2 equivalents) was added. Reaction mixture was stirred overnight at laboratory temperature. Mixture was extracted between H₂O and Petroleum ether, evaporated and quick chromatography was performed (Petroleum ether as mobile phase). Unpolar products at the start of the chromatography were separated and the polar part was concentrated in vacuum and dissolved in DMF (200 mg/ 1ml). Triphenylphosphine (5 equivalents) was added, and reaction mixture was stirred and heated for 100°C overnight. Crude mixture was concentrated in vacuum and purified with column chromatography (Chloroform: methanol gradient) to yield corresponding decyl triphenyl phosphine ether in the yield about 20%.

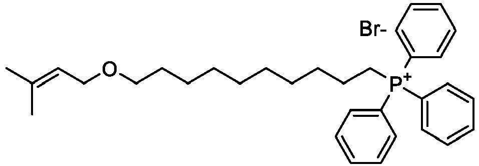

¹H NMR (700 MHz, Methanol-d4) δ 7.91 (td, J = 7.4, 1.8 Hz, 3H), 7.83 (ddd, J = 12.6, 8.4, 1.4 Hz, 6H), 7.80 - 7.75 (m, 6H), 5.40 - 5.26 (m, 1H), 3.97 (d, J = 6.9 Hz, 2H), 3.47 - 3.39 (m, 4H), 1.76 (s, 3H), 1.70 (s, 3H), 1.69 - 1.63 (m, 2H), 1.61 - 1.51 (m, 4H), 1.40 - 1.25 (m, 10H).

¹³C NMR (176 MHz, Methanol-d4) δ 136.58 , 134.86 (d, J = 3.0 Hz), 133.41 (d, J = 10.0 Hz), 130.11 (d, J = 12.6 Hz), 120.80 , 118.61 (d, J = 86.3 Hz), 69.70 , 66.69 , 30.19 (d, J = 16.2 Hz), 29.33, 29.09 , 28.92 , 28.63, 28.23, 25.83 , 24.51 , 22.11 (d, J = 4.4 Hz), 21.21 (d, J = 51.0 Hz), 16.63.

HRMS: calculated for C36H50P = 487.3129, found 487.3127.

### Example 2: (Z)-(18-hydroxyoctadec-9-en-1-yl)triphenylphosphonium bromide (Lab 205)

Starting (Z)-18-bromooctadec-9-en-1-ol (76 mg, 0.219 mmol) was dissolved in toluene (1.5 ml) and triphenylphosphine (576 mg, 2.19 mmol) was added. The reaction mixture was then stirred at 75 °C for 24 h. Solvent was evaporated and crude product was purified on column chromatography (CHCl₃→CHCl₃/MeOH/NH₃, 100/10/1). The product was a clear oil (106 mg, 79 %).

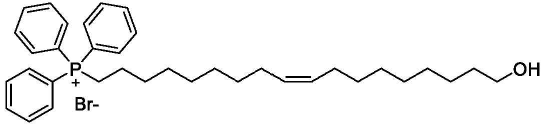

¹H NMR (700 MHz, Chloroform-d) δ 7.83 (ddd, *J =* 12.6, 7.1, 1.6 Hz, 6H), 7.78 (td, *J =* 7.5, 1.6 Hz, 3H), 7.69 (td, *J =* 7.8, 3.4 Hz, 6H), 5.31 (p, 2H), 3.80 - 3.72 (m, 2H), 3.61 (t, *J =* 6.7 Hz, 2H), 1.96 (dq, *J =* 14.3, 6.8 Hz, 4H), 1.65 - 1.57 (m, 3H), 1.57 - 1.51 (m, 2H), 1.34 - 1.15 (m, 19H). ¹³C NMR (176 MHz, Chloroform-d) δ 135.06 (d, *J =* 3.0 Hz, 3C), 133.80 (d, *J =* 10.0 Hz, 6C), 130.57 (d, *J* = 12.5 Hz, 6C), 130.02 , 129.94 , 118.56 (d, *J =* 85.8 Hz, 3C), 62.97 , 32.92 , 30.55 (d, *J =* 15.6 Hz), 29.79 , 29.71 , 29.53 , 29.50 , 29.39 , 29.30 , 29.24 , 29.17 , 27.25 , 27.18 , 25.89 , 22.78 (d, *J =* 4.6 Hz), 22.75 (d, *J =* 49.57).

HRMS calculated for C₃₆H₅₀OP⁺ 529.35938, found 529.36006.

### Example 3: (10-bromodecyl)tricyclohexylphosphonium bromide (Lab 210)

1,10-dibromodecane (7.490 g, 24.962 mmol) was dissolved in dry DMF (8 ml). Tricyclohexylphosphine (0.7 g, 2.496 mmol) was added, and the reaction mixture was stirred at 60 °C for 4 h and concentrated under reduced pressure. Crude product was purified on column chromatography on silicagel (CHCl₃/MeOH, 100/2→100/5). Product was clear oil (845 mg, 59%).

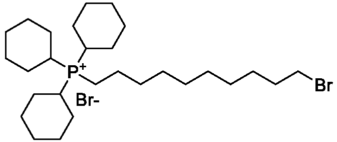

¹H NMR (700 MHz, Chloroform-d) δ 3.39 (t, *J =* 6.8 Hz, 2H), 2.65 (qt, *J =* 12.5, 2.9 Hz, 4H), 2.48 - 2.41 (m, 2H), 2.03 - 1.98 (m, 7H), 1.95 - 1.88 (m, 7H), 1.85 - 1.80 (m, 4H), 1.60 - 1.42 (m, 17H), 1.35 - 1.24 (m, 10H).

¹³C NMR (176 MHz, Chloroform-d) δ 34.31 , 32.81 , 31.37 (d, *J* = 13.8 Hz), 30.02 (d, *J =* 40.3 Hz, 3C), 29.31 (d, *J* = 7.4 Hz, 3C), 29.10 (d, *J* = 1.1 Hz), 28.73 , 28.14 , 27.39 (d, *J* = 3.9 Hz, 6C), 26.58 (d, *J =* 11.8 Hz, 6C), 25.57 , 25.56 , 22.97 (d, *J =* 5.4 Hz), 16.00 (d, *J =* 42.5 Hz).

MS (ESI) *m*/*z* (%) 499.6 (90), 501.6 (100), 502.6 (30).

### Example 4: Tricyclohexyl(16-hydroxyhexadecyl)phosphonium methansulfonate (Lab 161)

Methanesulphonic anhydride (148 mg, 0.8511 mmol) was added (in one portion) into suspension of 1,16-hexadecanediol (200 mg, 0.7738 mmol) in pyridine/dichloromethane mixture (1:1, 2 mL). The heterogeneous reaction mixture was stirred for 3 h at room temperature while turning brown. Reaction was quenched with methanol (2 mL) and washed between dichloromethane (2 × 40 mL) and aqueous citric acid (40 mL, 15%). The combined organic layer was concentrated under vacuum and tricyclohexylphosphine (1 g, solid) was added to the crude intermediate product. Phosphine was melted upon heating to 80°C, and the resulting mixture was stirred at 80°C for 48 h. The reaction mixture was cooled to room temperature, and a diethylether/petroleum ether mixture (1:1, 40 mL) was added. The mixture was stirred vigorously for two hours while cooled in an ice bath (4°C). The product precipitated on the walls of the round-bottom flask and the solvent was decanted off. Product was purified by column chromatography (10 mL silica, loaded in chloroform) by isocratic elution chloroform/methanol/ammonia 100:10:1. Product: Colorless, thick oil (16 mg)

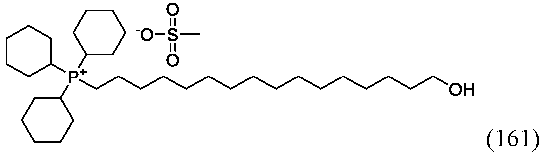

¹H NMR (700 MHz, Chloroform-d) δ 3.63 (t, *J =* 6.7 Hz, 2H), 2.97 - 2.76 (population of singlets, 3H), 2.60 - 2.49 (m, 3H), 2.33 (td, *J =* 12.2, 7.1 Hz, 2H), 2.02 - 1.90 (m, 12H), 1.89 - 1.79 (m, 6H), 1.55 (m, 4H),1.53 (m, 3H), 1.44 (dtd, *J =* 13.0, 9.9, 3.7 Hz, 6H), 1.36 -1.33 (m, 3H), 1.33-1.20 (m, 24H).

¹³C NMR (176 MHz, Chloroform-d) δ 62.94 ,45.80 - 41.45(mesylate singlets) 32.74, 31.21 (d, *J* = 13.6 Hz), 29.82 (d, *J =* 40.4 Hz), 29.58 , 29.53 (overlapping signals) , 29.47 , 29.45 , 29.37 , 29.36 , 29.29 , 29.25 , 29.17 , 28.99 , 28.50 , 28.35 , 27.12 (d, *J =* 3.9 Hz), 26.48 (d, *J =* 11.7 Hz), 25.83 , 25.71 , 25.49 , 25.48 , 25.41 , 25.24 , 25.22 , 22.73 , 22.70 , 15.46 (d, *J =* 42.6 Hz).

HRMS: calculated for C₃₄H₆₆OP = 521.48458, found = 521.4856.

### Example 5: (16-hydroxyhexadecyl)triphenylphosphonium bromide (Lab 162)

16-bromohexadecanol (494 mg, 1.537 mmol) was dissolved in DMF (10 ml) and triphenylphosphine (423 mg, 1.613 mmol) was added. The reaction mixture was then stirred and heated to 85°C for 24 h. The crude material was allowed to cool to laboratory temperature and added dropwise to an ice cold and vigorously stirred diethyl ether solution (200 ml). The precipitate was decanted, concentrated under vacuum, and purified by column chromatography (chloroform: methanol) to obtain the product in 65% yield (580 mg).

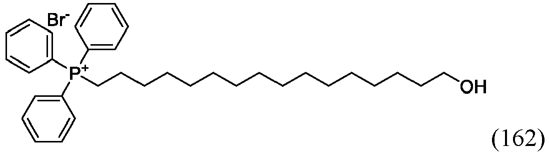

¹H NMR (500 MHz, Methanol-*d*₄) δ 7.91 (tt, *J* = 7.1, 1.8 Hz, 3H), 7.86 - 7.80 (m, 6H), 7.80 - 7.74 (m, 6H), 3.55 (t, *J =* 6.7 Hz, 2H), 3.46 - 3.37 (m, 2H), 3.37 (s, 6H), 1.74 - 1.62 (m, 2H), 1.56 (dt, *J =* 13.8, 7.1 Hz, 4H), 1.41 - 1.24 (m, 24H).

¹³C NMR (126 MHz, Methanol-*d*₄) δ 134.87 (d, *J =* 2.9 Hz), 133.39 (d, *J =* 9.6 Hz), 130.11 (d, *J* = 12.4 Hz), 118.60 (d, *J =* 85.9 Hz), 61.59, 32.26 , 30.26 , 30.13 , 29.34 , 29.32 , 29.29 , 29.20 , 29.14 , 28.98, 28.48 , 22.13 (d, *J =* 4.5 Hz).

### Example 6: (8-((9-hydroxynonyl)oxy)octyl)triphenylphosphonium chloride (Lab 197)

8-((9-hydroxynonyl)octyl 4-methylbenzenesulfonate (600 mg, 1.357 mmol) was dissolved in dry DMF (3 ml) under argon atmosphere. Triphenyl phosphine (3.2 g, 12.210 mmol) was added and reaction mixture was stirred for 5 h at 100 °C. Solvent was evaporated under reduced pressure and crude product was purified on column chromatography on silica gel (pure CHCl₃ then CHCl₃/MeOH/NH₃, 100/5/0.5→ 100/10/1). Desired phosphine (280 mg, 30%) was placed on ionex (30 ml) and converted into Cl-. Chromatography on silica gel was repeated (CHCl₃/MeOH, 100/2.5→100/10). Product was clear oil (180 mg).

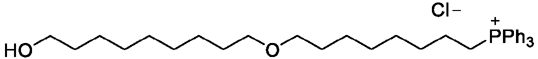

¹H NMR (600 MHz, Chloroform-d) δ 7.87 - 7.81 (m, 6H), 7.80 - 7.75 (m, 3H), 7.72 - 7.67 (m, 6H), 3.81 (qd, J = 8.2, 7.3, 4.8 Hz, 2H), 3.60 (t, J = 6.8 Hz, 2H), 3.34 (dt, J = 12.3, 6.6 Hz, 4H), 1.64 - 1.57 (m, 4H), 1.56 - 1.45 (m, 6H), 1.34 - 1.18 (m, 12H).

¹³C NMR (151 MHz, Chloroform-d) δ 135.02 (d, J = 3.0 Hz), 133.80 (d, J = 10.0 Hz), 130.56 (d, J = 12.5 Hz), 118.64 (d, J = 85.7 Hz), 70.94 , 70.86 , 62.94, 32.92, 30.44 (d, J = 15.6 Hz), 29.78 , 29.76 , 29.62 , 29.44 , 29.41 , 29.31 , 29.14 , 26.21 , 26.18 , 25.87 , 22.78 (d, J = 4.6 Hz), 22.54 (d, J = 49.5 Hz). MS (ESI) m/z (%) 533.4 (100, M), 534.4 (37), 535.4 (6).

HRMS calculated for C₃₅H₅₀O₂P⁺: 533.35429, found: 533.35494. IR 3326.10, 2830.08, 1582.64, 1447.83, 1019.82 cm⁻¹.

### Example 7: Screening experiment for IC50 of control and senescent cells

In vitro, we tested the effect of ATPP derivatives (described above) on untreated HEK cells or treated with 100uM BrdU for 8 days to induce senescence. Control (200 000 per well, cells reached 80% confluence) and senescent cells (80 000 per well, cells reached 80% confluence) were treated with different doses of ATPP derivates diluted in ethanol (0.08, 0.15, 0.31, 0.48, 0.62, 0.93, 1.25, 2.5, 5µM) for 48h, their viability were measured using the WST-1 assay and IC50 (the concentration at which cell viability reaches 50%) was determined (Table 1). From all tested ATPP derivatives, we found four (Lab161, Lab162, Lab205, Lab210) that eliminates senescent cells at lower concentrations than control cells, which provides therapeutic window, where compound will eliminate senescent cells without affecting normal cells.

**Table 1**

| **Compound** | **IC50 (µM)** | |
|---|---|---|
| | **Control** | **Senescent** |
| Lab161 | 1,3 | 0,64 |
| Lab162 | 0,67 | 0,35 |
| Lab167 | 0,2 | 0,21 |
| Lab197 | 0,26 | 0,29 |
| Lab205 | 0,68 | 0,41 |
| Lab210 | 1,05 | 0,46 |

### Example 8: In vivo experiment - effect of ATPP compounds on senescent cells elimination

To prove the role of ATPP derivatives in the elimination of senescent cells *in vivo,* mice with diet-induced obesity and prediabetes fed a high-fat diet (HFD) for 8 months were used as a model of age-related disease. Mice fed a standard diet (SD) for eight months were used as control. Mice were treated intraperitoneally with Lab 162, Lab205, Lab210 (2 mg/kg of body weight dissolved in 4% EtOH in corn oil for each treatment), or vehicle (corn oil, CO) twice per week for four weeks. The presence of senescent cells in kidney (Table 2) and liver (Table 3) measured by expression of *p21* as a marker using RT-qPCR was determined. The data indicate that Lab 162 and Lab210 significantly decrease senescence in both, kidney and liver of HFD mice. Data are expressed as mean ± SEM; *p<0.05, **p<0.01, ***p<0.005 relative to HFD+CO.

**Table 2**

| | **p21 Kidney (fold change)** | |
|---|---|---|
| | **Avarage** | **SD** |
| SD+CO | 1,0 ** | 0,3 |
| SD+Lab162 | 0,6 ** | 0,4 |
| SD+Lab205 | 1,1 * | 0,3 |
| SD+Lab210 | 1,1 * | 0,3 |
| HFD+CO | 1,7 | 0,6 |
| HFD+Lab162 | 1,0 * | 0,3 |
| HFD+Lab205 | 1,5 | 0,6 |
| HFD+Lab210 | 1,1 * | 0,2 |

**Table 3**

| | **p21 Liver (fold change)** | |
|---|---|---|
| | **Avarage** | **SD** |
| SD+CO | 1,0 *** | 0,8 |
| SD+Lab162 | 0,6 *** | 0,6 |
| SD+Lab205 | 1,1 ** | 0,8 |
| SD+Lab210 | 0,7 *** | 0,5 |
| HFD+CO | 2,9 | 1,2 |
| HFD+Lab162 | 0,9 ** | 0,7 |
| HFD+Lab205 | 2,4 | 1,1 |
| HFD+Lab210 | 1,5 * | 0,7 |

### Example 9: In vivo experiment - effect of ATPP compounds on weight and adipose tissue reduction

To see the effect of ATPP derivatives on obesity we used the model of C57BL/6 male mice fed with HFD for 8 months featuring significantly increased body weight and white adipose tissue mass compared to mice fed SD. Mice were treated intraperitoneally with Lab 162, Lab205, Lab210 (2mg/kg of body weight dissolved in 4% EtOH in corn oil for each treatment) or vehicle (corn oil; CO) twice per week for four weeks. Decrease in body weight (Table 4) expressed as delta body weight (the difference between the final and initial weight) and weight of white adipose tissue (WAT; composed of subcutaneous, visceral and perirenal adipose tissue) was observed for all tested compounds (Table 5). Data are expressed as mean ± SEM; *p<0.05; **p<0.01; ***p<0.005 relative to HFD+CO.

**Table 4**

| | **Delta body weight (g)** | |
|---|---|---|
| | **Avarage** | **SD** |
| SD+CO | 1,59 | 2,28 |
| SD+Lab162 | -1,28 | 2,45 |
| SD+Lab205 | -3,05 | 2,57 |
| SD+Lab210 | -1,58 | 1,70 |
| HFD+CO | 0,02 | 4,31 |
| HFD+Lab162 | -8,2 * | 10,34 |
| HFD+Lab205 | -5,99** | 5,15 |
| HFD+Lab210 | -5,85 ** | 2,88 |

**Table 5**

| | **WAT weight (g)** | |
|---|---|---|
| | **Avarage** | **SD** |
| SD+CO | 1467,6 *** | 194,3 |
| SD+Lab162 | 1305,8 *** | 469,6 |
| SD+Lab205 | 1121,2 *** | 383,9 |
| SD+Lab210 | 1322,2 *** | 278,8 |
| HFD+CO | 8108,8 | 1588,6 |
| HFD+Lab162 | 6042,8 ** | 1179,6 |
| HFD+Lab205 | 7094,8 * | 1047,2 |
| HFD+Lab210 | 6761,9 ** | 765,7 |

### Example 10: In vivo experiment - effect of ATPP compounds on glucose tolerance

To determine the effect of ATPP derivatives on type 2 diabetes mellitus, we used a model of C57BL/6 male mice fed with HFD for 8 months, featuring significantly increased glycemia compared to mice fed SD. Mice were treated intraperitoneally with Lab 162, Lab205, Lab210 (2mg/kg of body weight dissolved in 4% EtOH in corn oil for each treatment) or vehicle (corn oil; CO) twice per week for four weeks. Increased glucose uptake from blood measured by oral glucose tolerance test (oGTT) was observed for all tested compounds (Table 6). Data are expressed as mean ± SEM; **p<0.01; ***p<0.005 relative to HFD+CO.

**Table 6**

| | **oGTT (AUC)** | |
|---|---|---|
| | **Avarage** | **SD** |
| SD+CO | 1861,5 *** | 262,2 |
| SD+Lab162 | 1843,4 *** | 189,6 |
| SD+Lab205 | 1770,2 *** | 203,3 |
| SD+Lab210 | 1860,9 *** | 214,8 |
| HFD+CO | 2363,3 | 297,5 |
| HFD+Lab162 | 1897,5 ** | 296,7 |
| HFD+Lab205 | 1952,7 ** | 202,8 |
| HFD+Lab210 | 2024,6 ** | 131,7 |

### Example 11: In vivo experiment - effect of ATPP compounds on insulin resistance

To see the effect of ATPP derivates on insulin resistance we used the model of C57BL/6 male mice fed with HFD for 8 months featuring hyperinsulinemia compared to mice fed SD. Mice were treated intraperitoneally with Lab 162, Lab205, Lab210 (2mg/kg of body weight dissolved in 4% EtOH in corn oil for each treatment) or vehicle (corn oil; CO) twice per week for four weeks. Decreased level of insulin in serum was observed for all tested compounds (Table 7). Data are expressed as mean ± SEM; **p<0.01; ***p<0.005 relative to HFD+CO.

**Table 7**

| | **Insulin (pg/ml)** | |
|---|---|---|
| | **Avarage** | **SD** |
| SD+CO | 931,8 *** | 214,9 |
| SD+Lab162 | 1100,0 *** | 861,5 |
| SD+Lab205 | 646,9 *** | 156,2 |
| SD+Lab210 | 772,5 *** | 424,0 |
| HFD+CO | 2752,8 | 1193,0 |
| HFD+Lab162 | 1602,0 ** | 1236,7 |
| HFD+Lab205 | 1937,1 ** | 733,9 |
| HFD+Lab210 | 1693,5 ** | 967,0 |

### Example 12: In vivo experiment - effect of ATPP compounds on accumulation of lipids in liver

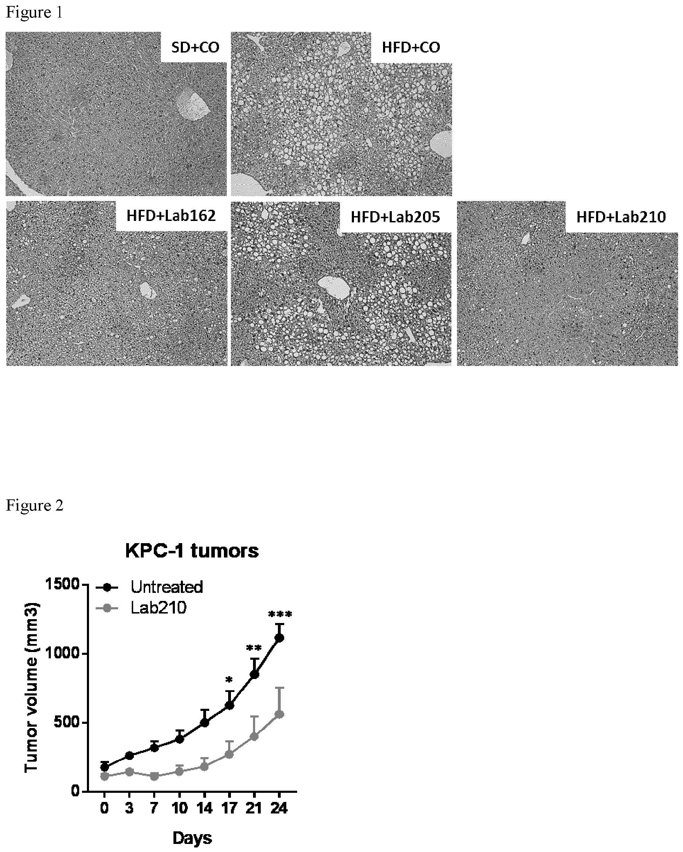

To see the effect of ATPP derivates on liver lipid accumulation leading to development of non-alcohol fatty liver diseases we used the model of C57BL/6 male mice fed with HFD for 8 months featuring liver steatosis compared to mice fed SD. Mice were treated intraperitoneally with Lab162, Lab205, Lab210 (2mg/kg of body weight dissolved in 4% EtOH in corn oil for each treatment) or vehicle (corn oil; CO) twice per week for four weeks. Decreased level of triacylglycerol (TAG) in liver (Table 8) accompanied with decreased "ballooning" caused by lipid accumulation (Figure 1) was observed for Lab162 and Lab210. Data are expressed as mean ± SEM; *p<0.05; **p<0.01; ***p<0.005 relative to HFD+CO.

**Table 8**

| | **TAG Liver (µmol/g)** | |
|---|---|---|
| | **Avarage** | **SD** |
| SD+CO | 25,7 *** | 9,8 |
| SD+Lab162 | 43,1 *** | 11,2 |
| SD+Lab205 | 18,1 *** | 5,9 |
| SD+Lab210 | 27,7 *** | 8,3 |
| HFD+CO | 532,8 | 89,7 |
| HFD+Lab162 | 309,9 ** | 40,5 |
| HFD+Lab205 | 527,0 | 117,5 |
| HFD+Lab210 | 412,8 * | 119,7 |

### Example 13: In vivo experiment - effect of ATPP compounds on KCP-1 pancreatic tumor cells

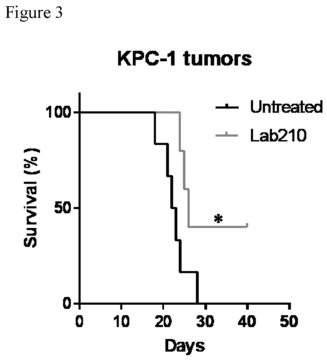

Dysfunctional pancreatic β-cells occurring in metabolic disorders produce increased levels of pro-inflammatory molecules and increase the development of fibrotic tissue, which increases the risk of cancer. Since tumor cells as well as senescent cells are strongly dependent on functional mitochondria and both cell types are known to increase mitochondrial potential pointing to specific targeting with ATPP compounds, the effect of ATPP derivatives on tumor growth was tested. KPC-1 pancreatic tumor cells (1×10⁶) were grafted subcutaneously into syngeneic C57BL6/J mice (untreated n=6; Lab210, n=5). When tumors reached approximately 100 mm³, mice were treated twice per week by intraperitoneal administration of Lab210 (3 mg/kg) dissolved in 4% EtOH in corn oil or vehicle (corn oil; CO). Significantly decreased tumor growth (Figure 2) and prolonged survival (Figure 3) were observed in mice treated with Lab210. Data are expressed as mean ± SEM; *p<0.05; **p<0.01; ***p<0.005.

### Example 14: In vivo experiment - effect of ATPP compounds on pancreatic fibrosis

To see the effect of ATPP salts on pancreatic fibrosis, a common comorbidity observed in subjects with obesity and diabetes, which can progress to pancreatitis, we used the model of C57BL/6 male mice fed with HFD for 8 months featuring significantly increased pancreatic fibrosis compared to mice fed SD. Mice were treated intraperitoneally with Lab205, Lab210 (2mg/kg of body weight dissolved in 4% EtOH in corn oil for each treatment) or vehicle (corn oil; CO) twice per week for four weeks. Decreased expression of fibronectin 1 (FN1), a marker of fibrosis, was observed for both tested compounds (Table 9). Data are expressed as mean ± SEM; *p<0.05; relative to HFD+CO.

**Table 9**

| | **FN1 Pancreas (fold change)** | |
|---|---|---|
| | **Avarage** | **SD** |
| SD+CO | 1 * | 0,04 |
| SD+Lab205 | 1,47 | 1,30 |
| SD+Lab210 | 0,58 * | 0,60 |
| HFD+CO | 3,34 | 1,30 |
| HFD+Lab205 | 0,13 * | 0,04 |
| HFD+Lab210 | 0,18 * | 0,08 |

### Example 15: In vivo experiment - effect of ATPP respiratory diseases

To see the effect of ATPP salts on respiratory diseases, we used the model of C57BL/6 male mice fed with HFD for 8 months featuring increased pulmonary fibrosis compared to mice fed SD, which can progress into chronic obstructive pulmonary disease. Mice were treated intraperitoneally with Lab205 or Lab210 (2mg/kg of body weight dissolved in 4% EtOH in corn oil for each treatment) or vehicle (corn oil) twice per week for four weeks. Decreased presence of fibrotic tissue in lungs was observed for both compounds (Table 10). Data are expressed as mean ± SEM.

**Table 10**

| | **% of fibrotic tissue (lungs)** | |
|---|---|---|
| | **Avarage** | **SD** |
| SD+CO | 4,14 | 0,80 |
| SD+Lab205 | 3,80 | 0,85 |
| SD+Lab210 | 4,16 | 1,41 |
| HFD+CO | 8,39 | 6,19 |
| HFD+Lab205 | 3,63 | 0,55 |
| HFD+Lab210 | 4,73 | 0,36 |

## Claims

1. Phosphonium compounds of general formula I 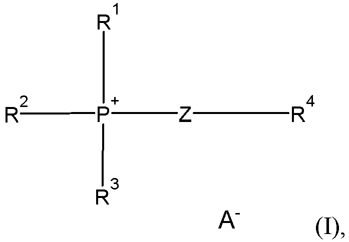
for use in the treatment of metabolic diseases;
wherein
R¹, R², R³ are independently selected from the group consisting of phenyl, benzyl, and cyclohexyl; Z is a linear alkylene, alkenylene or alkynylene chain with the number of carbon atoms in the range of from 6 to 20, wherein one or more -CH₂- groups may optionally be substituted with one or more O atoms;
R⁴ is selected from the group consisting of H, OH, halogen atom, and (C1 to C10)alkoxy group, which may optionally be substituted with one or more -OH groups;
A⁻ is a pharmaceutically acceptable anion.

2. Phosphonium compounds of general formula I for use according to claim 1, wherein A⁻ is selected from the group comprising Cl⁻, Br⁻, I⁻, carbonate, bicarbonate, nitrate, sulphate, phosphate, hexafluorophosphate, tetrafluoroborate, acetate, ascorbate, aspartate, benzenesulfonate, benzoate, besylate, bitartrate, camsylate, citrate, decanoate, edetate, esylate, formiate, fumarate, gluceptate, gluconate, glutamate, glycolate, hexanoate, hydroxynaphthoate, isethionate, lactate, lactobionate, malate, maleate, mandelate, mesylate, methyl sulfate, methansulfonate, mucate, napsylate, octanoate, oleate, oxalate, pamoate, pantothenate, polygalacturonate, propionate, salicylate, stearate, succinate, tartrate, teoclate, and tosylate.

3. Phosphonium compounds of general formula I for use according to claim 1 or 2, wherein all of R¹, R² and R³ are the same, preferably selected from phenyl and cyclohexyl.

4. Phosphonium compounds of general formula I for use according to claim 1, 2 or 3, wherein R⁴ is selected from the group consisting of H, OH, and halogen atom.

5. Phosphonium compounds of general formula I for use according to any one of claims 1 to 4, wherein Z is a linear alkylene linker of formula -(CH₂)ₙ-, wherein n is an integer in the range of from 6 to 20, wherein one or more -CH₂- groups may optionally be substituted with one or more O atoms.

6. Phosphonium compounds of general formula I for use according to any one of claims 1 to 5, wherein Z is a linear (C6 to C20)alkenylene linker, preferably containing one double bond, wherein one or more -CH₂- groups may optionally be substituted with one or more O atoms.

7. Phosphonium compounds of general formula I for use according to any one of claims 1 to 6, wherein the phosphonium compounds of general formula I are selected from:
(18-hydroxyoctadec-9-en-1-yl)triphenylphosphonium;
(10-bromodecyl)tricyclohexylphosphonium;
Tricyclohexyl(16-hydroxyhexadecyl)phosphonium methansulfonate
(16-hydroxyhexadecyl)triphenylphosphonium.

8. Phosphonium compounds of general formula I for use according to any one of claims 1 to 7, wherein the metabolic diseases are selected from the group comprising type 2 diabetes mellitus, obesity, cardiorenal diseases, respiratory diseases, neurodegenerative diseases, and cancer.

9. Phosphonium compounds of general formula for use according to any one of claims 1 to 8, wherein the metabolic diseases are selected from the group comprising obesity, type 2 diabetes mellitus, liver steatosis, pancreatic tumour, pancreatic fibrosis, and/or pulmonary fibrosis.

10. Phosphonium compounds of general formula I for use according to any one of the preceding claims 1 to 9, wherein the metabolic diseases are selected from the group comprising type 2 diabetes mellitus, obesity, chronic/diabetic kidney disease, metabolic dysfunction-associated fatty liver disease, metabolic dysfunction-associated steatohepatitis, atherosclerosis, cardiac ischemia, heart failure with preserved ejection fraction, heart failure with reduced ejection fraction, idiopatic pulmonary disease, chronic obstructive pulmonary disease, diabetic neuropathy, and pancreatic cancer.

11. Phosphonium compounds of general formula I as defined in any one of the preceding claims 1 to 7 for use in a prophylactic treatment, preferably for the prevention of metabolic diseases in individuals suffering from obesity or in overweight individuals.

12. Phosphonium compounds of general formula I as defined in any one of the preceding claims 1 to 7, for use in a prevention of obesity in overweight individuals.

13. Use of phosphonium compounds of general formula I as defined in claims 1 to 7 as a food supplement to reduce overweight and/or to improve overweight-induced metabolic complications in overweight individuals.

14. A pharmaceutical composition **characterized in that** it contains at least one phosphonium compound of general formula I, as defined in any one of the preceding claims 1 to 7, as the active pharmaceutical ingredient, and at least one pharmaceutically acceptable excipient.
